# EUROPEAN PATENT SPECIFICATION

(11) **EP 3 488 897 B1**
(45) Date of publication and mention of the grant of the patent: **07.10.2020**
(21) Application number: 19152404.0
(22) Date of filing: 04.03.2015
(51) Int. Cl.: A61N 1/05, A61B 90/00

(54) **MEDICAL DEVICE HAVING ECHOGENIC FEATURES**
MEDIZINISCHE VORRICHTUNG MIT ECHOGENEN MERKMALEN
DISPOSITIF MÉDICAL DOTÉ DE CARACTÉRISTIQUES ÉCHOGÈNES

(30) Priority: 28.03.2014 US 201461972070 P
(43) Date of publication of application: 29.05.2019
(62) Divisional of application: 15712721.8
(73) Proprietor: Gyrus ACMI, Inc. d/b/a Olympus Surgical Technologies America, Southborough, MA 01772 (US)
(72) Inventor: SHUMAN, Brandon J., Kirkland, WA 98033 (US); GONZALEZ, Hugo Xavier, Woodinville, WA 98072 (US)
(74) Representative: Noack, Andreas

(56) References cited:
- US-A1- 2011 046 619
- US-A1- 2012 059 247
- US-A1- 2012 059 308
- US-A1- 2012 271 163
- US-A1- 2013 190 609
- US-B1- 6 416 510

## Description

### FIELD

The present disclosure relates to medical devices, and more particularly, to systems and devices for insertion into a patient's anatomy. US-A1-2013/190609 and US-A1-2011/046619 disclose a medical device system in accordance with the preamble of claim 1. US-A1-2012/271163 discloses a lead fixation element for securing an implantable lead to adjacent body tissue wherein the fixation element includes a fixation helix adapted to rotate and translate out from within an interior space of a collar. US-A1-2012/059308 discloses a catheter comprising a coil extending along the length of a sheath in a helical pattern.

### BACKGROUND

The statements in this section merely provide background information related to the present disclosure and may or may not constitute prior art.

Lung nodules, lesions, tumors, and other cancerous or pre-cancerous regions of tissue in the lung may be difficult to treat with invasive surgical techniques, with attendant complications such as excessive bleeding, infection risk, air leaks, pneumothorax, and other such issues. In particular, regions deep in the lung may be difficult to access using conventional methods, further increasing the difficulty of treatment.

Electrical ablation, in particular radiofrequency electrical ablation, has been used in the treatment of tumors and other masses present in solid tissues such as the liver. Use of such techniques, however, entails some attendant complications and difficulties. First, the use of conventional electrical ablation probes requires piercing into the thoracic cavity and into the lung, with a consequent high likelihood of pneumothorax, excessive bleeding and other complications. Moreover, these transthoracic ablation probes are rigid and may not be able to reach certain areas, such as tumors located near central organs or vasculature.

While there have been some attempts to pursue radiofrequency electrical ablation via bronchoscopes inserted into airways, these attempts are limited by the confines of the airway passage and the reach of the bronchoscope and accordingly may not be able to position the probes and/or deliver sufficient energy to treat tissue such as lung nodules adequately.

In addition, visualization and localization of the tissue region to be treated may present challenges, especially for tissue regions deep in the lung. Likewise, such visualization within the body may present challenges for procedures in tissue in other areas of the body outside of the lung region. Ultrasound techniques do not always provide for sufficient viewing of the medical devices used. As a result, in ablation procedures, surgeons may be unsure of whether adequate ablation has occurred.

### SUMMARY

The present disclosure provides a device having echogenic features for visualization of the device under ultrasound. The device forms a coil having echogenic features. A medical device system may be used having an outer catheter and a movable medical device disposed therein, wherein the medical device comprises echogenic features.

Accordingly, pursuant to one aspect of the invention, there is contemplated a medical device system for insertion into a patient's anatomy in accordance with claim 1.

Pursuant to another aspect, there is disclosed a surgical electrode for insertion into a patient's anatomy. The surgical electrode includes a coil defining a plurality of helical turns. At least one echogenic feature is defined by the coil for allowing visualization of the coil when the coil is inserted into the patient's anatomy. The surgical electrode is configured to deliver an energy to a tissue.

The invention may be further characterized by one or any combination of the features described herein, such as: the introducer tube is configured to serve as a first electrode; the medical device is configured to serve as a second electrode; the first and second electrodes are configured to deliver an energy to a tissue; the introducer tube has a piercing tip; the medical device is a coil; the echogenic feature(s) include a plurality of holes formed through the coil; each hole has a diameter in the range of about 0.001 inch to about 0.003 inch; the coil defines a plurality of helical turns; each helical turn forms a hole; each hole is aligned along an axis; the axis is disposed along one side of the coil; the echogenic feature includes an open continuous channel formed along each helical turn; the echogenic feature includes a pointed edge along the coil; the pointed edge has two sides that intersect each other at an obtuse angle; the echogenic feature includes a channel formed in the coil and having a plurality of outlet holes formed in the coil; the channel is configured to deliver a fluid through the channel and to dispense the fluid from the plurality of outlet holes; and the echogenic feature(s) include a plurality of irregularities formed in the coil.

Further aspects, advantages and areas of applicability will become apparent from the description provided herein. It should be understood that the description and specific examples are intended for purposes of illustration only and are not intended to limit the scope of the present disclosure.

### DRAWINGS

The drawings described herein are for illustration purposes only and are not intended to limit the scope of the present disclosure in any way.
FIG. 1A is schematic side view of a medical device system including a medical device in a first position, in accordance with the principles of the present disclosure;
FIG. 1B is a schematic side view of the medical device system of FIG. 1A, with the medical device in a second position, in accordance with the principles of the present disclosure;
FIG. 2 is a perspective view of a medical device, in accordance with the principles of the present disclosure;
FIG. 3 is a perspective view of another medical device, in accordance with the principles of the present disclosure;
FIG. 4A is a perspective view of yet another medical device, in accordance with the principles of the present disclosure;
FIG. 4B is a cross-sectional view of the medical device of FIG. 4A taken along the lines 4B-4B, in accordance with the principles of the present disclosure;
FIG. 5 is a side perspective view of still another medical device, in accordance with the principles of the present disclosure;
FIG. 6 is a side schematic view of still another medical device, in accordance with the principles of the present disclosure; and
FIG. 7 is a side schematic view of yet another medical device, in accordance with the principles of the present disclosure.

### DETAILED DESCRIPTION

The following description is merely exemplary in nature and is not intended to limit the present disclosure, application, or uses.

The present disclosure provides a medical device having echogenic features for visualization of the medical device under ultrasound. The medical device may be, for example, a coil having echogenic features. A medical device system may be used having an outer catheter and a movable medical device disposed therein, wherein the medical device comprises echogenic features.

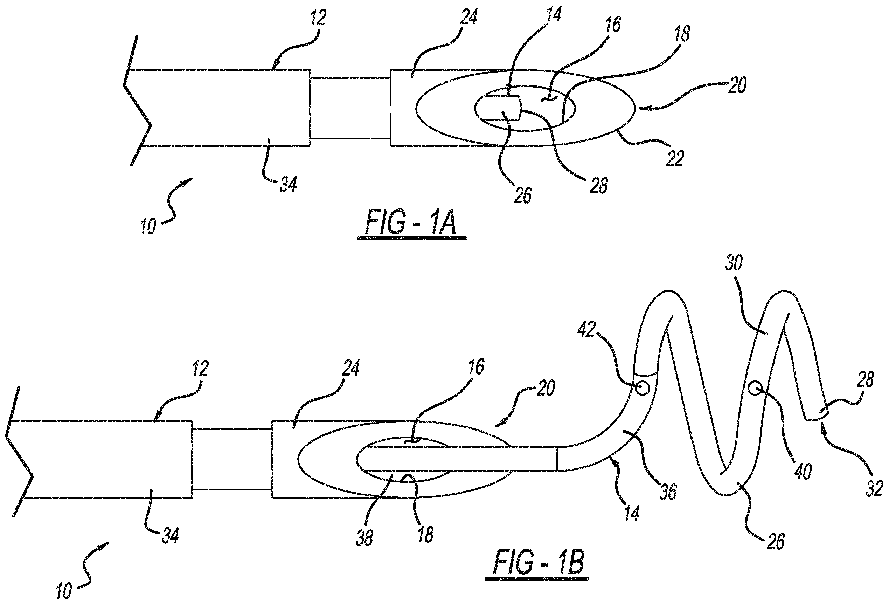

With reference to the figures, wherein like numerals indicate like components, and specifically with reference to FIGS. 1A-1B, an example of a medical device system in accordance with the principles of the present disclosure is illustrated and generally designated at 10. The medical device system 10 is configured to be inserted into a patient's anatomy, for example, for treatment of tissue, such as pulmonary tissue. The medical device system 10 may be configured to be used in thoracoscopic, laparoscopic, transcutaneous, and/or percutaneous procedures, by way of example. In some variations, the medical device system 10 may be inserted into a bronchoscope, such as a BF-P180 bronchoscope manufactured by Olympus and/or an EBUS® scope manufactured by Olympus. In some configurations, the medical device system 10 may be inserted into an airway so that the medical device system 10 reaches or is placed proximate a region of tissue to be treated.

The medical device system 10 may include an introducer tube 12 and a medical device 14. The introducer tube 12 defines a lumen 16 therein and an opening 18 at a distal end 20 of the introducer tube 12. The introducer tube 12 may be in the form of a hollow needle having a piercing tip 22. The piercing tip 22 may be used to pierce through an airway wall, a tumor, or other tissue, for example, but without limitation. As such, the piercing tip 22 may have a sharp edge or end that may pierce, perforate, or penetrate into or through tissue.

FIG. 1A illustrates a first position of the medical device 14. In the first position, the medical device 14 is disposed within the lumen 16 of the introducer tube 12. The medical device 14 is movable within the introducer tube 12 between the first position and a second position (illustrated in FIG. 1B). When it is desired to extend the medical device 14 from the introducer tube 12, the medical device 14 may be pushed or otherwise moved through lumen 16 and out of the opening 18 of the distal end 20 of the introducer tube 12. In the second position (see FIG. 1B), the medical device 14 extends at least partially beyond the distal end 20 of the introducer tube 12.

The introducer tube 12 may bear, or serve as, a first electrode 24. For example, the introducer tube 12 may itself be the first electrode 24, or the first electrode 24 may be attached to the introducer tube 12. In FIGS. 1A-1B, the first electrode 24 is the needle end of the introducer tube 12. The medical device 14 may bear, or serve as, a second electrode 26. For example, the medical device 14 may itself be the second electrode 26, or the second electrode 26 may be attached to the medical device 14. In the illustrated example, the medical device 14 is itself the second electrode 26; however, the second electrode is given its own numeral 26 when referred to as the second electrode 26. This is because, it should be understood, that in other embodiments, the medical device 14 may be a non-energized device.

Each of the first and second electrodes 24, 26 has its own electrical lead (not shown) to connect the respective electrode 24, 26 to a power source (not shown). The power source may be connected to the leads via wires and the like. As such, the power source is operable to deliver power to the first electrode 24 and/or the second electrode 26 through the leads (not shown). Thus, the power source may be configured to deliver energy to a region of tissue via the medical device system 10, which includes one or more of the first and second electrodes 24, 26.

In some configurations, the power source (not shown) comprises a source of electric or electromagnetic power. Other sources of energy, alone or in combination, can be used and energy can be delivered to the tissue via the power source (not shown) and/or the medical device system 10. Such power sources can include direct current treatment, cryo treatment (including cryoablation), microwave, laser, and/or photodynamic treatment, by way of example.

In some configurations, the power source may be configured to deliver electric power at varied frequencies. In some configurations, the power source may be configured to deliver radiofrequency (RF) energy in a range of between about 3 KHz and about 300 GHz. In some configurations, the range may be between about 100 KHz and about 500 KHz. In some configurations, the range may be between about 300 KHz and about 400 KHz. In some configurations, the power source may be configured to deliver power in a range of between about 5 watts and about 40 watts, or between about 7 watts and about 25 watts, or between about 8 watts and about 13 watts, by way of example.

In some configurations, the power level may be set by the user or operator, and the resulting voltage and current will vary with that setting. In some configurations, the voltage and current may vary between the ranges of about 20 VAC and about 60 VAC and between about 0.1 ampere and about 1 ampere. In some configurations, the energy delivered to a 1 cm diameter volume treatment site may be between about 8 KJ and about 13 KJ, depending on the type of tissue.

In some configurations, the medical device system 10 may act to heat or ablate tissue via RF energy. Tissues such as tumors (especially lung nodules) or other tissue masses may be treated with energy so as to heat the cells therein to ablate, kill, burn, heat, or denature the cells. The tissue may not necessarily need to be heated so as to kill the component cells, but may be heated enough to modify the cells so as to become non-malignant or otherwise benign. This may also be achieved by cooling, such as by cryoablation.

In some configurations, energy such as RF energy may be delivered by a single electrode, for example, only one of the electrodes 24, 26. In such configurations, the electrical field may emanate away from the electrode as a single point source. A surface pad may serve as a second electrode. In other configurations, energy may be delivered via a bipolar electrode assembly. In such configurations, the electrical field may emanate between two respective poles formed by the first and second electrodes 24, 26. Thus, the first and second electrodes 24, 26 are configured to apply a current, or deliver an energy, to a tissue.

Additional electrodes may be used for multipolar electrode treatment. In the illustrated example, the needle tip serves as the first electrode 24, however, in other configurations, more than two electrodes may be disposed at or near the distal end 20 of the introducer tube 12. In other configurations, multiple medical device systems 10 may be used.

When the medical device 14 (second electrode 26) is disposed within the first electrode 24, only the introducer tube 12 may need to be sufficiently strong to pierce through an airway wall (e.g., with the piercing tip 22), rather than needing two separate electrodes possessing sufficient strength or rigidity or being provided with piercing tips arranged to puncture the airway wall. On the other hand, in some configurations, the medical device 14 may be provided with, or attached to, a piercing end 28. The piercing end 28 can be used to penetrate into tissue being treated (e.g., a lung nodule). In some configurations, the piercing end 28 may be sheathed by the first electrode 24. In some configurations, the piercing end 28 may be exposed when the medical device 14 is extended from the first electrode 24.

The medical device 14 may have a main body portion 30 terminating in the piercing end 28, by way of example, if the optional piercing end 28 is included. In the illustrated example, the main body portion 30 is also the second electrode 26 portion of the medical device 14. In some embodiments, the main body portion 30 may be flexible, but relatively straight except for a slight curve at a distal tip thereof, when disposed in the lumen 16 of the introducer tube 12, as illustrated in FIG. 1A. When the medical device 14 is extended from the introducer tube 12 and into the second position, the main body portion 30 may curve or form a spiral or coil. In some configurations, the main body portion 30 can be formed or arranged to take a helical or spiral form. Such configurations may be preferable because they may induce an eddy current into the tissue being treated, in addition to the joule heating resulting from a resistance of the tissue being treated. In some configurations, the main body portion 30 is flexible, and adapts a helical, spiral, or coiled configuration once extended away from the distal end 20 of the introducer tube 12. In some configurations, the main body portion 30 may comprise a superelastic material (e.g., Nitinol) and the main body portion 30 can change shapes. Other materials may also be used, including conductive polymers and bundles of multiple wires (such as a cable), which may in some configurations provide for greater elasticity.

As explained above, in some configurations, the main body portion 30 can be manufactured at least in part from a shape-memory material, such as Nitinol. In some such configurations, the main body portion 30 may have an austenite configuration above body temperature that forms a coil or bend. The main body portion 30 may be loaded into the introducer tube 12 in the martensite configuration and in a straighter form, such that heating of the main body portion 30 (e.g., due to electric current passing through the main body portion 30 or by contact of the main body portion 30 with warmer body tissue) causes the main body portion 30 to convert to the austenite configuration and form a bend or coil. In some configurations, the main body portion 30 may be deployed into tissue while still straight, followed by subsequent heating to cause it to change shape. In some configurations, the main body portion 30 may be heated as it is inserted into the tissue (e.g., as it exits the opening 18 of the distal end 20 of the introducer tube 12) so that the main body portion 30 begins to bend or coil as it is deployed. In still other configurations, the main body portion 30 may simply return to a coil shape once deployed by virtue of a spring force in its superelastic material. It is contemplated that deployment of the main body portion 30 of the medical device 14 into a bent or coiled configuration may result in the electrode assembly entering a tissue locking position such that the distal end 32 of the medical device 14 is maintained in position with respect to the treatment area of interest. Such a locking position may be maintained during breathing or heat treatment. It is contemplated that the medical device 14 may go through multiple deployments from the introducer tube 12 without moving the introducer tube 12.

Preferably, the main body portion 30 may be shaped as a coil with a pitch in a range of between about 0.1 mm to about 2 mm, and preferably about 1 mm, by way of example. The major diameter of the coil may measure between about 2 mm and about 10 mm, and preferably between about 3 mm and about 4 mm. The coil may also comprise between about 0.5 total helical turns and about 5 total helical turns, and preferably between about 1.5 total helical turns and about 3 total helical turns. The wire diameter that may be used to manufacture the coil may measure between about 0.010 inches and about 0.020 inches, and preferably about 0.015 inches.

In some configurations, at least one of the first electrode 24 and the second electrode 26 includes an insulating layer 34, 36. In some configurations, for example, an insulating layer 36 can be positioned between the first electrode 24 and the second electrode 26. In some configurations, the insulating layer can be formed on an inner surface 38 of the first electrode 24 and/or on an outer surface of the second electrode 26 (see, e.g., insulating layer 36 on the outer surface of the medical device 14). Such a placement of the insulating layer(s) can serve to reduce the likelihood of short circuiting between the electrodes 24, 26 while improving the use of bipolar or multipolar ablation configurations. In some configurations, the first electrode 24 comprises an insulating layer 34 that terminates proximally of the distal extremity of the first electrode 24. In some such configurations, the insulating layer 34 can be partially removed or stripped to expose one or more conductive surfaces of the first electrode 24.

In some configurations, insulating materials may be lubricious. Lubricious insulating materials can improve the ability of the electrodes 24, 26 to move relative to each other. Any suitable insulating material may be used to overlay at least a portion of the one or more electrodes 24, 26. In some configurations, the insulating material may comprise a polymeric material. For example, PTFE, fluorinated ethylene propylene, high density polyethylene, polyethylene, and/or other suitable insulating materials may be used. In some embodiments, the use of saline (e.g., saline conductive gel) can reduce friction between the electrodes 24, 26. In some embodiments, one or more surfaces of the electrodes 24, 26 can be coated with a ceramic powder.

When using bipolar or multipolar electrical ablation, in particular RF ablation, the first and second electrodes 24, 26 can be used to concentrate the energy being delivered to the surrounding tissue into a zone roughly bounded by these electrodes 24, 26. The degree of extension of the second electrode 26 into the tissue permits the user to modulate the amount and area of energy being directed into the surrounding tissue. In some configurations, the first and second electrodes 24, 26 can be configured to limit the range of relative extension. For example, the range of relative extension between the first and second electrodes 24, 26 can be predetermined based upon the size of the nodule or other area of interest to be treated. In some embodiments, the deployed distance between the first and second electrodes 24, 26 is configured to be approximately equal to the depth of the nodule or other area of interest into which the first and/or second electrodes 24, 26 are deployed. In some configurations, the extent to which the first and second electrodes 24, 26 can move relative to each other in the distal and/or proximal directions is approximately equal to the distance required to move the proximal end of the second electrode 26 from the stored position (first position) to the deployed position (second position). In some embodiments, the extent to which the first and second electrodes 24, 26 can move relative to each other in the distal and/or proximal directions is greater than the deployed distance between the first and second electrodes 24, 26 due, for example, to the second electrode 26 being stored in a relative straight configuration, or first position, within the lumen 16 of the introducer tube 12 prior to deployment into the second position.

The medical device 14 defines at least one echogenic feature thereon for allowing visualization of the medical device 14 upon insertion of the medical device 14 in the patient's anatomy. In FIG. 1B, two echogenic features are included, in the form of two holes 40, 42 formed in the medical device 14. The first hole 40 is formed through the electrode 26 or main body portion 30 of the medical device 14, and the second hole 42 is formed through the insulated portion 36, in this example.

The echogenic features, or holes 40, 42, increase the medical device's 14 echogenicity, or ability to be seen under ultrasound when inserted into a patient's anatomy. Thus, the user can see where the medical device 14 has been deployed using, for example, standard ultrasound bronchoscopy.

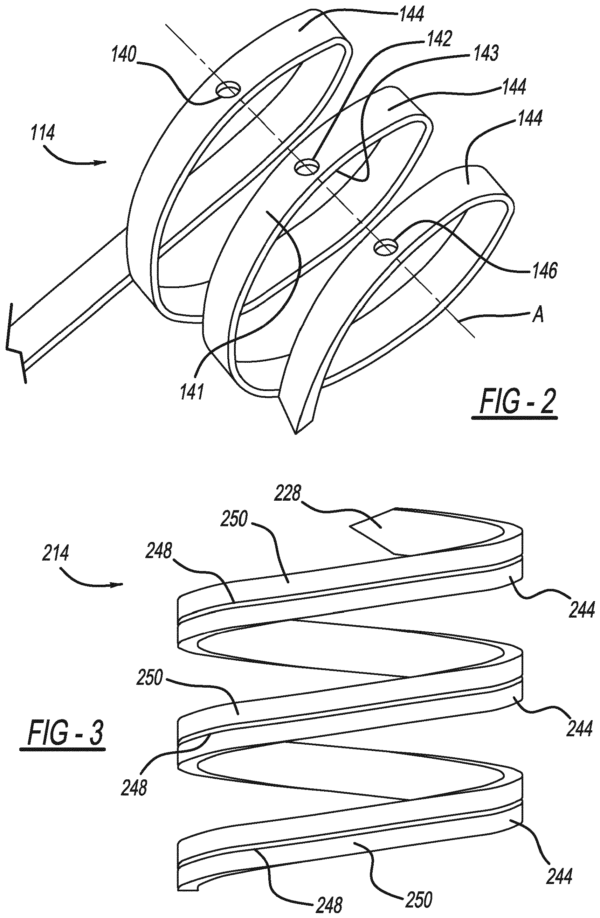

Referring now to FIG. 2, another example of a medical device 114 is illustrated. It should be understood that the medical device 114 may be used with the introducer tube 12 in the same way as the medical device 14, if desired. In addition, the medical device 114 may serve as an electrode, as described above. All other details not described with respect to FIG. 2 may be similar or the same as the features described with respect to the example in FIGS. 1A-1B.

The medical device 114 forms a coil once deployed within the patient's anatomy. The medical device 114 has a flat, ribbon shape and defines a plurality of helical turns 144. In this example, three helical turns 144 are illustrated, however, a greater or lower number of helical turns 144 could be used alternatively. A plurality of echogenic features are included on the medical device 114 in the form of holes 140, 142, 146. Each hole 140, 142, 146 is formed through one of the helical turns 144. Each hole 140, 142, 146 extends through front 141 and rear 143 surfaces of the medical device 114. Like the echogenic features 40, 42 described above, the holes 140, 142, 146 aid in the visibility of the medical device 114 under ultrasound. The holes 140, 142, 146 may also serve to capture a small amount of tissue during ablation. The tissue can then be analyzed after the device 114 is retracted into the introducer 12 and removed from the body.

The holes 140, 142, 146 each have a diameter in the range of about 0.001 inch to about 0.003 inch. In some examples, the holes 140, 142, 146 each have a diameter of about 0.002 inch. In addition, though the holes 140, 142, 146 are illustrated as being circular in shape, they could have other shapes, such as an ovular, square, or rectangular shape, by way of example. In one variation, the holes 140, 142, 146 could be ovular with dimensions of about 0.002 inch to about 0.005 inch. In the illustrated example, each hole 140, 142, 146 is disposed and aligned along an axis A, and the axis A is disposed along one side of the coil formed by the deployed medical device 114. Thus, the holes 140, 142, 146 are disposed along a face of the coil for visualization by ultrasound.

Although three holes 140, 142, 146 are illustrated, a greater or fewer number of holes 140, 142, 146 may be used. In addition, in some configurations, the holes 140, 142, 146 may not be aligned along the axis A. Instead, the holes 140, 142, 146 could be in other places on the coil of the medical device 114. For example, the holes 140, 142, 146 could be placed in 90° intervals around the helical turns 144 of the coil of the medical device 114.

Referring now to FIG. 3, another example of a medical device 214 is illustrated. It should be understood that the medical device 214 may be used with the introducer tube 12 in the same way as the medical device 14, if desired. In addition, the medical device 214 may serve as an electrode, as described above. All other details not described with respect to FIG. 3 may be similar or the same as the features described with respect to the example in FIGS. 1A-1B.

The medical device 214 forms a coil illustrated in a deployed configuration. The medical device 214 has a flat, ribbon shape and defines a plurality of helical turns 244. In this example, three helical turns 244 are illustrated, however, a greater or lower number of helical turns 244 could be used alternatively. The medical device 214 may have a piercing distal end 228.

The medical device 214 has an echogenic feature in the form of a continuous open channel 248, or groove formed continuously along the outer side 250 of the coil formed by the medical device 214 once deployed, extending along each of the helical turns 244. In other embodiments, the groove 250 could be discontinuous, however. Fluid or bubbles, such as saline, may be injected and wicked along the continuous channel 248, which may aid in the visualization of the medical device 214, once deployed. The fluid may be echogenic. In one example, the fluid may contain a fluorescent substance. The interface of air and saline may produce reflection of ultrasound waves. Micro-bubbles may also be used to reflect ultrasound waves. For example, a bubble having a size of about 0.002 inch could be used. Generally, the bubbles vibrate at the same ultrasonic frequency as the medical device 214. The bubbles, however, reflect back the ultrasonic wave more efficiently than the surface of the medical device 214. Glass microbeads may also be used in a fluid and injected along the open channel 248. For example, glass microbeads having a diameter of about 0.002 inch may be used. The continuous channel 248 may be filled with an adhesive or polymer, such as, for example, cyanoacrylate or urethane, that has a foaming agent in it. In such an arrangement, the foaming agent encases microbubbles that, in turn, increases the echogenicity of the medical device 214.

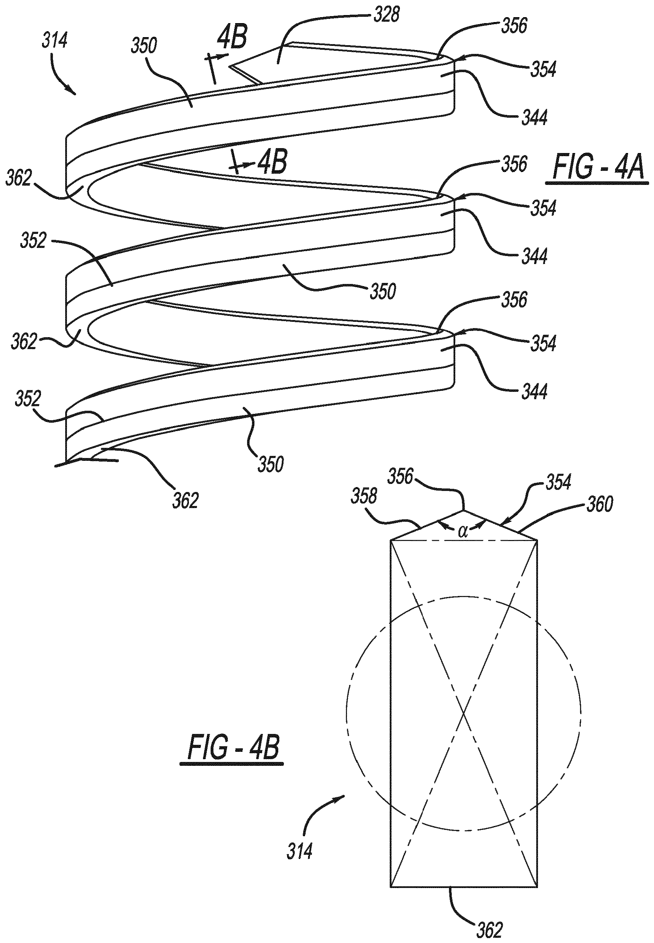

Referring now to FIGS. 4A-4B, another example of a medical device 314 is illustrated. It should be understood that the medical device 314 may be used with the introducer tube 12 in the same way as the medical device 14, if desired. In addition, the medical device 314 may serve as an electrode, as described above. All other details not described with respect to FIGS. 4A-4B may be similar or the same as the features described with respect to the example in FIGS. 1A-1B.

The medical device 314 forms a coil once deployed within the patient's anatomy. The medical device 314 has a flat, ribbon shape and defines a plurality of helical turns 344. In this example, three helical turns 344 are illustrated, however, a greater or lower number of helical turns 344 could be used alternatively. The medical device 314 may have a piercing distal end 328.

The medical device 314 has an echogenic feature in the form of a score line 352 formed continuously along the outer side 350 of the coil formed by the medical device 314 once deployed, extending along each of the helical turns 344. In other embodiments, the score line 352 could be discontinuous, however. The score line 352 may be similar to the open channel 248 described above. Fluid or bubbles, such as saline, may be injected and wicked along the score line 352, which may aid in the visualization of the medical device 314, once deployed. The fluid may be echogenic, for example, it may contain a fluorescent substance. The interface of air and saline may produce reflection of ultrasound waves. Micro-bubbles may also be used to reflect ultrasound waves. For example, a bubble having a size of about 0.002 inch could be used. Generally, the bubbles vibrate at the same ultrasonic frequency as the medical device 314. The bubbles, however, reflect back the ultrasonic wave more efficiently than the surface of the medical device 314. Glass microbeads may also be used in a fluid and injected along the score line 352. For example, glass microbeads having a diameter of about 0.002 inch may be used. The score line 352 may be filled with an adhesive or polymer, such as, for example, cyanoacrylate or urethane, that has a foaming agent in it. In such an arrangement, the foaming agent encases microbubbles that, in turn, increases the echogenicity of the medical device 314.

One or more edges of the coil formed by the medical device 314 may have a pointed edge 354 disposed continuously along the coil formed by the medical device 314. The pointed edge 354 has a first side 358 and a second side 360 that meet in a point 356 along the pointed edge 354. The first and second sides 358, 360 meet at an angle α. In the illustrated example, the angle α is an obtuse angle. However, it should be understood that α could have other dimensions; for example, α could be a right angle or an acute angle. The pointed edge 354 is an echogenic feature for aiding in the visualization of the medical device 314 under ultrasound.

Though the point 356 is only shown along the pointed edge 354, it should be understood that a similar point could be constructed along the opposite plain edge 362, if desired.

Though FIG. 4A illustrates the use of both a score line 352 and a pointed edge 354 as echogenic features, it should be understood that either the score line 352 or the pointed edge 354 could be used. It should also be understood that any of the echogenic features disclosed herein could be combined with each other.

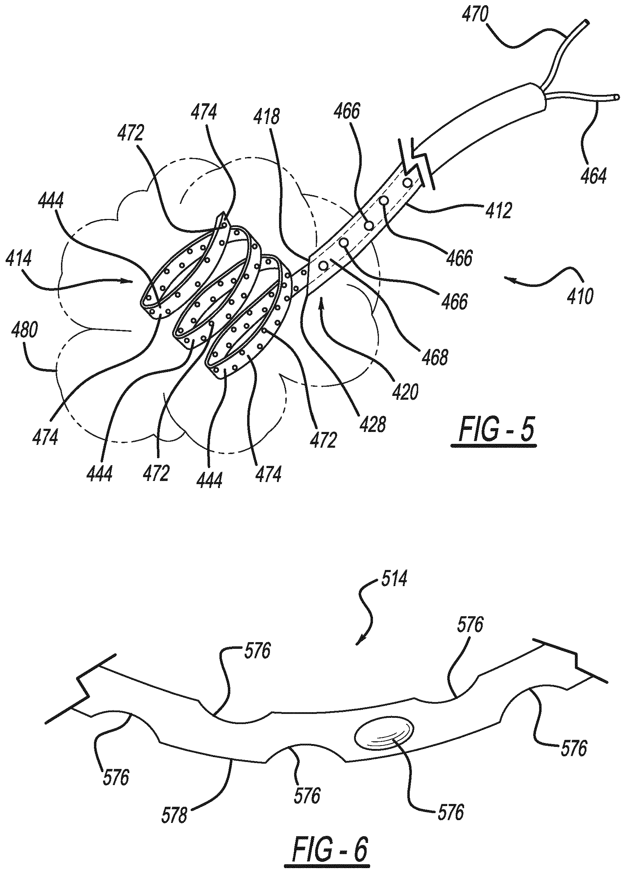

Referring now to FIG. 5, another example of a medical device system 410, including a medical device 414 and introducer tube 412, is illustrated. The medical device 414 is shown in a deployed second position extending from the introducer tube 412 and into a tumor 480. It should be understood that the medical device 414 may be moved within the lumen of the introducer tube 412 between first and second positions, as described above with respect to FIGS. 1A-1B. The medical device 414 and the introducer tube 412 may serve as a monopolar or bipolar electrodes, as described above. All other details not described with respect to FIG. 2 may be similar or the same as the features described with respect to the example in FIGS. 1A-1B.

The introducer tube 412 may have a piercing tip 422 and an opening 418 disposed at a distal end 420 of the introducer tube 412. A tube 464 has a lumen is in fluid communication with a plurality of outlet holes 466 formed in an outer side 468 of the introducer tube 412. Fluid, such as saline, bubbles, or fluid containing glass beads, as described above, may be injected through the tube 464 and out of the outlet holes 466. Such fluid and outlet holes 466 may aid in the visualization of the introducer tube 412 under ultrasound.

The medical device 414 forms a coil once deployed within the patient's anatomy, as illustrated in FIG. 1B. The medical device 414 defines a plurality of helical turns 444. In this example, three helical turns 444 are illustrated, however, a greater or lower number of helical turns 444 could be used alternatively.

A coil tube 470 has a channel formed therethrough. The coil tube 470 extends through the introducer tube 412 and into a channel formed in the medical device 414 through each of the helical turns 444. A channel formed in the coil tube 470 communicates with the channel formed in the medical device 414. The channel in the medical device 414 communicates with a plurality of outlet holes 472 formed through the outer side(s) 474 of the medical device 414. Outlet holes 472 are formed in each of the helical turns 444.

Like the echogenic features described above, the channel and outlet holes 472 aid in the visibility of the medical device 414 under ultrasound. In this case, fluid is injected through the outlet holes 472 in the medical device 414 and the outlet holes 466 in the introducer tube 412. Like the holes 140, 142, 146 described above, the outlet holes 466, 472 may each have a diameter in the range of about 0.001 inch to about 0.003 inch. In some examples, the outlet holes 466, 472 may each have a diameter of about 0.002 inch. In addition, though the outlet holes 466, 472 may have a variety of shapes, such as circular, ovular, square, or rectangular, by way of example.

Fluid or bubbles, such as saline, may be injected through each of the outlet holes 472, 466, originating in the channels in the tube 464 and coil tube 470, which may aid in the visualization of the medical device 414, once deployed. The fluid may be echogenic, for example, it may contain a fluorescent substance. The interface of air and saline may produce reflection of ultrasound waves. Micro-bubbles may also be used reflect ultrasound waves. For example, a bubble having a size in the range of about 0.0002 inch to about 0.002 inch could be used. Generally, the bubbles vibrate at the same ultrasonic frequency as the medical device 314. The bubbles, however, reflect back the ultrasonic wave more efficiently than the surface of the medical device 314. Glass microbeads may also be used in a fluid and injected through the outlet holes 466, 472. For example, glass microbeads having a diameter of about 0.002 inch may be used. The holes 476, 466 may be filled with an adhesive or polymer, such as, for example, cyanoacrylate or urethane, that has a foaming agent in it. In such an arrangement, the foaming agent encases microbubbles that, in turn, increases the echogenicity of the medical device 314.

In sum, FIG. 6 illustrates an example having a channel formed by the medical device 414, having outlet holes 472, wherein fluid flows from the channel through the outlet holes 472.

Referring now to FIG. 6, another example of a medical device 514 is illustrated. It should be understood that the medical device 514 may be used with the introducer tube 12 in the same way as the medical device 14, if desired. In addition, the medical device 514 may serve as an electrode, as described above. All other details not described with respect to FIG. 6 may be similar or the same as the features described with respect to the example in FIGS. 1A-1B.

In the illustrated embodiment, only a portion of the medical device 514 is shown, but it should be understood that the medical device 514 may form a coil once deployed within the patient's anatomy. A plurality of echogenic features is included on the medical device 514 in the form of irregularities 576. The irregularities 576 may be in the form of variations in the surface 578 of the device 514, the irregularities may include scores, scratch marks, or scooped out portions formed in the surface 578 of the device 514, by way of example. For example, the irregularities 576 may extend into the medical device 514 at different angles and locations to reflect sound waves.

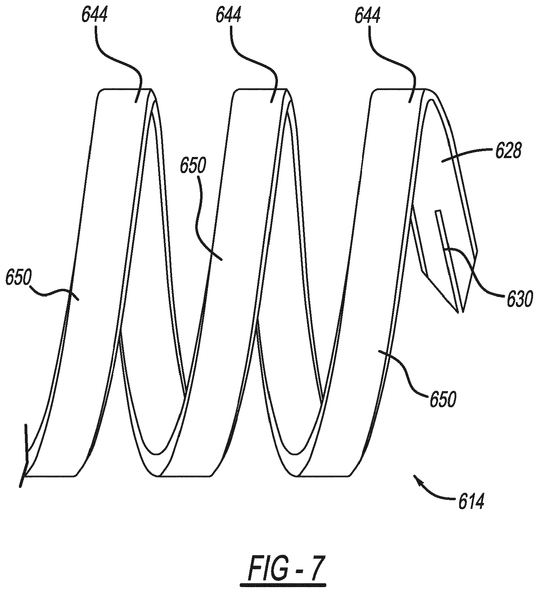

Referring now to FIG. 7, another example of a medical device 614 is illustrated. It should be understood that the medical device 614 may be used with the introducer tube 12 in the same way as the medical device 14, if desired. In addition, the medical device 614 may serve as an electrode, as described above. All other details not described with respect to FIG. 7 may be similar or the same as the features described with respect to the example in FIGS. 1A-1B.

The medical device 614 forms a coil illustrated in a deployed configuration. The medical device 614 has a flat, ribbon shape and defines a plurality of helical turns 644 with an outer surface 650. In this example, three helical turns 644 are illustrated, however, a greater or lower number of helical turns 644 could be used alternatively. The medical device 614 may have a piercing distal end 628.

The medical device 614 has an echogenic feature in the form of a slot 630 at the distal end 628. Fluid or bubbles, such as saline, may be injected and wicked along the slot 630, which may aid in the visualization of the medical device 614, once deployed. The fluid may be echogenic. In one example, the fluid may contain a fluorescent substance. The interface of air and saline may produce reflection of ultrasound waves. Micro-bubbles may also be used to reflect ultrasound waves. For example, a bubble having a size of about 0.002 inch could be used. Generally, the bubbles vibrate at the same ultrasonic frequency as the medical device 614. The bubbles, however, reflect back the ultrasonic wave more efficiently than the surface of the medical device 614. Glass microbeads may also be used in a fluid and injected along the slot 630. For example, glass microbeads having a diameter of about 0.002 inch may be used. The slot 630 may be filled with an adhesive or polymer, such as, for example, cyanoacrylate or urethane, that has a foaming agent in it. In such an arrangement, the foaming agent encases microbubbles that, in turn, increases the echogenicity of the medical device 614.

The description of the invention is merely exemplary in nature. For example, variations in the various figures can be combined with each.

For example, it should be understood that the embodiments disclosed herein are merely examples, and variations may occur without departing from the spirit and scope of the invention, as defined by the claims. The specific illustrations are examples and are not meant to limit the invention in any way.

The preferred embodiment of the present invention has been disclosed. A person of ordinary skill in the art would realize, however, that certain modifications would come within the teachings of this invention. Therefore, the following claims should be studied to determine the true scope and content of the invention.

Any numerical values recited in the above application include all values from the lower value to the upper value in increments of one unit provided that there is a separation of at least 2 units between any lower value and any higher value. As an example, if it is stated that the amount of a component or a value of a process variable such as, for example, temperature, pressure, time and the like is, for example, from 1 to 90, preferably from 20 to 80, more preferably from 30 to 70, it is intended that values such as 15 to 85, 22 to 68, 43 to 51, 30 to 32 etc. are expressly enumerated in this specification. For values which are less than one, one unit is considered to be 0.0001, 0.001, 0.01 or 0.1 as appropriate. These are only examples of what is specifically intended and all possible combinations of numerical values between the lowest value and the highest value enumerated are to be considered to be expressly stated in this application in a similar manner.

Unless otherwise stated, all ranges include both endpoints and all numbers between the endpoints, the use of "about" or "approximately" in connection with a range apply to both ends of the range. Thus, "about 20 to 30" is intended to cover "about 20 to about 30", inclusive of at least the specified endpoints.

The term "consisting essentially of" to describe a combination shall include the elements, ingredients, components or steps identified, and such other elements ingredients, components or steps that do not materially affect the basic and novel characteristics of the combination.

The use of the terms "comprising" or "including" describing combinations of elements, ingredients, components or steps herein also contemplates embodiments that consist essentially of the elements, ingredients, components or steps.

The term "consisting essentially of" to describe a combination shall include the elements, ingredients, components or steps identified, and such other elements ingredients, components or steps that do not materially affect the basic and novel characteristics of the combination.

The use of the terms "comprising" or "including" describing combinations of elements, ingredients, components or steps herein also contemplates embodiments that consist essentially of the elements, ingredients, components or steps.

## Claims

1. A medical device system for insertion into a patient's anatomy, the medical device system comprising:
an introducer tube (12) defining a lumen (14) therein, the introducer tube defining an opening (18) at a distal end (20) of the introducer tube (12);
a medical device (514) disposed within the lumen (14) of the introducer tube (12) in a first position, the medical device (514) being movable within the introducer tube (12) between the first position and a second position, wherein in the second position, the medical device extends at least partially beyond the distal end (20) of the introducer tube (12), the medical device (514) defining at least one echogenic feature (576) thereon for allowing visualization of the medical device (514) when the medical device (514) is inserted into the patient's anatomy in the second position;
**characterized in that**:
the medical device (514) forms a coil once deployed within the patient's anatomy;
the medical device (514) is stored in a relative straight configuration within the lumen (14) of the introducer tube (12) in the first position and has a coil portion and a straight portion in the second position, and
the coil defines a plurality of helical turns (244), the at least one echogenic feature comprising a plurality of irregularities (576) formed in the coil.

2. The medical device system of claim 1, wherein the irregularities (576) are variations in a surface (578) of the medical device (514).

3. The medical device system of claim 1, wherein the irregularities (576) include scores formed in the surface (578) of the medical device (514).

4. The medical device system of claim 1, wherein the irregularities (576) include scratch marks formed in the surface (578) of the medical device (514).

5. The medical device system of claim 1, wherein the irregularities (576) include scooped out portions formed in the surface (578) of the medical device (514).

6. The medical device system of claim 1 wherein the irregularities (576) extend into the medical device (514) at different angles and locations to reflect sound waves.

7. The medical device system of claim 1, the introducer tube (12) being configured to serve as a first electrode (24), and the medical device (514) being configured to serve as a second electrode (26), the first and second electrodes (24, 26) being configured to deliver an energy to a tissue, the introducer tube (12) having a piercing tip (22).

## Patentansprüche

1. Medizinvorrichtungssystem für die Einführung in die Anatomie eines Patienten, wobei das Medizinvorrichtungssystem Folgendes umfasst:
einen Einführschlauch (12), der ein Lumen (14) darin definiert, wobei der Einführschlauch eine Öffnung (18) an einem distalen Ende (20) des Einführschlauchs (12) definiert;
eine Medizinvorrichtung (514), die innerhalb des Lumens (14) des Einführschlauchs (12) in einer ersten Position angeordnet ist, wobei die Medizinvorrichtung (514) innerhalb des Einführschlauchs (12) zwischen der ersten Position und einer zweiten Position beweglich ist, wobei sich die Medizinvorrichtung in der zweiten Position wenigstens teilweise über das distale Ende (20) des Einführschlauchs (12) hinaus erstreckt, wobei die Medizinvorrichtung (514) wenigstens ein echogenes Merkmal (576) darauf definiert, um eine Visualisierung der Medizinvorrichtung (514) zu ermöglichen, wenn die Medizinvorrichtung (514) in der zweiten Position in die Anatomie des Patienten eingeführt wird;
**dadurch gekennzeichnet, dass**:
die Medizinvorrichtung (514) eine Spule ausbildet, sobald sie innerhalb der Anatomie des Patienten entfaltet ist;
die Medizinvorrichtung (514) in einer relativ geraden Konfiguration innerhalb des Lumens (14) des Einführschlauchs (12) in der ersten Position gelagert ist und einen Spulenabschnitt und einen geraden Abschnitt in der zweiten Position aufweist, und
die Spule mehrere schraubenförmige Windungen (244) definiert, wobei das wenigstens eine echogene Merkmal mehrere Unregelmäßigkeiten (576) umfasst, die in der Spule ausgebildet sind.

2. Medizinvorrichtungssystem nach Anspruch 1, wobei die Unregelmäßigkeiten (576) Variationen in einer Oberfläche (578) der Medizinvorrichtung (514) sind.

3. Medizinvorrichtungssystem nach Anspruch 1, wobei die Unregelmäßigkeiten (576) Kerben beinhalten, die in der Oberfläche (578) der Medizinvorrichtung (514) ausgebildet sind.

4. Medizinvorrichtungssystem nach Anspruch 1, wobei die Unregelmäßigkeiten (576) Kratzspuren beinhalten, die in der Oberfläche (578) der Medizinvorrichtung (514) ausgebildet sind.

5. Medizinvorrichtungssystem nach Anspruch 1, wobei die Unregelmäßigkeiten (576) abgetragene Abschnitte beinhalten, die in der Oberfläche (578) der Medizinvorrichtung (514) ausgebildet sind.

6. Medizinvorrichtungssystem nach Anspruch 1, wobei sich die Unregelmäßigkeiten (576) unter verschiedenen Winkeln und Stellen in die Medizinvorrichtung (514) erstrecken, um Schallwellen zu reflektieren.

7. Medizinvorrichtungssystem nach Anspruch 1, wobei der Einführschlauch (12) konfiguriert ist, um als eine erste Elektrode (24) zu dienen, und die Medizinvorrichtung (514) konfiguriert ist, um als eine zweite Elektrode (26) zu dienen, wobei die erste und die zweite Elektrode (24, 26) konfiguriert sind, um eine Energie an ein Gewebe abzugeben, wobei der Einführschlauch (12) eine Stichspitze (22) aufweist.

## Revendications

1. Système de dispositif médical à insérer dans l'anatomie d'un patient, le système de dispositif médical comprenant :
un tube introducteur (12) définissant une lumière (14) à l'intérieur, le tube introducteur définissant une ouverture (18) à une extrémité distale (20) du tube introducteur (12) ;
un dispositif médical (514) disposé dans la lumière (14) du tube introducteur (12) dans une première position, le dispositif médical (514) étant mobile à l'intérieur du tube introducteur (12) entre la première position et une seconde position, dans lequel dans la seconde position, le dispositif médical s'étend au moins partiellement au-delà de l'extrémité distale (20) du tube introducteur (12), le dispositif médical (514) définissant au moins une caractéristique échogène (576) sur celui-ci pour permettre la visualisation du dispositif médical (514) lorsque le dispositif médical (514) est inséré dans l'anatomie du patient dans la seconde position ;
**caractérisé en ce que** :
le dispositif médical (514) forme une bobine une fois déployé dans l'anatomie du patient ;
le dispositif médical (514) est stocké dans une configuration rectiligne relative à l'intérieur de la lumière (14) du tube introducteur (12) dans la première position et présente une partie bobine et une partie droite dans la seconde position, et la bobine définit une pluralité de spires hélicoïdales (244), l'au moins une caractéristique échogène comprenant une pluralité d'irrégularités (576) formées dans la bobine.

2. Système de dispositif médical selon la revendication 1, dans lequel les irrégularités (576) sont des variations d'une surface (578) du dispositif médical (514).

3. Système de dispositif médical selon la revendication 1, dans lequel les irrégularités (576) comportent des entailles formées dans la surface (578) du dispositif médical (514).

4. Système de dispositif médical selon la revendication 1, dans lequel les irrégularités (576) comportent des rayures formées dans la surface (578) du dispositif médical (514).

5. Système de dispositif médical selon la revendication 1, dans lequel les irrégularités (576) comportent des parties évidées formées dans la surface (578) du dispositif médical (514).

6. Système de dispositif médical selon la revendication 1, dans lequel les irrégularités (576) s'étendent dans le dispositif médical (514) à différents angles et emplacements pour réfléchir les ondes sonores.

7. Système de dispositif médical selon la revendication 1, le tube introducteur (12) étant conçu pour servir de première électrode (24), et le dispositif médical (514) étant conçu pour servir de seconde électrode (26), les première et seconde électrodes (24, 26) étant conçues pour fournir une énergie à un tissu, le tube introducteur (12) ayant une pointe de perçage (22).
